# EUROPÄISCHE PATENTSCHRIFT

(11) **EP 2 443 991 B1**
(45) Veröffentlichungstag und Bekanntmachung des Hinweises auf die Patenterteilung: **17.08.2016**
(21) Anmeldenummer: 10013823.9
(22) Anmeldetag: 20.10.2010
(51) Int. Cl.: A61B 3/103, A61B 3/13, G02B 21/00, A61B 90/00

(54) **Operationsmikroskop mit Vorrichtung zur intraoperativen Refraktionsmessung**
Operation microscope with device for intra-operative refraction measurement
Microscope d'opération doté d'un dispositif pour la mesure de réfraction intra-opératoire

(43) Veröffentlichungstag der Anmeldung: 25.04.2012
(73) Patentinhaber: Möller-Wedel GmbH & Co. KG, 22880 Wedel (DE)
(72) Erfinder: Koetke, Jochen, 22339 Hamburg (DE)
(74) Vertreter: Glawe, Delfs, Moll

(56) Entgegenhaltungen:
- WO-A1-00/40142
- WO-A1-2007/028564
- DE-A1- 4 310 561
- DE-A1-102005 031 496
- DE-B3-102007 026 044

## Beschreibung

Die Erfindung betrifft ein Operationsmikroskop für die Augenchirurgie, insbesondere für Katarakt-Operationen. Das Operationsmikroskop umfasst eine Frontlinse, ein Vergrößerungssystem und einen Einblick. Mit dem Operationsmikroskop wird ein Beobachtungsstrahlengang definiert, der in der Augenlinsenebene fokussierbar ist.

Operationsmikroskope werden seit Jahrzehnten in der Augenchirurgie eingesetzt und sind unverzichtbares Instrument für die präzise chirurgische Therapie, beispielsweise von Katarakt (Grauer Star), Cornea- und Netzhauterkrankungen. Operationsmikroskope sind weit verbreitet und ihr typischer optischer und mechanischer Aufbau ist der Fachwelt bekannt.

Der häufigste augenchirurgische Eingriff ist die Kataraktoperation: Die eingetrübte natürliche Linse des Auges wird chirurgisch entfernt und durch eine künstliche, sogenannte Intraokularlinse ersetzt, um das Sehvermögen des Patienten wieder herzustellen. Um die korrekte Brechkraft der Intraokularlinse zu bestimmen, werden die erforderlichen biometrischen Daten des Patientenauges vor der Operation mit diagnostischen Geräten gemessen. Anhand empirischer Formeln, die von einem Modellaufbau des Auges ausgehen, wird aus den Daten die benötigte Dioptrienstärke der Intraokularlinse berechnet. Diesen empirischen Formeln liegen Modellannahmen zugrunde. Sind diese nicht oder nur teilweise erfüllt, besitzt die implantierte Intraokularlinse nicht die korrekte Dioptrienzahl. Es kann sich dann postoperativ herausstellen, dass die erzielte Refraktion des Auges inakzeptabel weit vom gewünschten Ergebnis abweicht. In Extremfällen muss die Intraokularlinse ausgetauscht werden. All das ist für den Patienten belastend und unerfreulich.

Dies kann insbesondere bei Patienten auftreten, die sich vorher einmal zur Visuskorrektur einer sogenannten LASIK unterzogen haben. Denn bei diesem Verfahren werden mittels eines Lasers Teile der Hornhaut abgetragen, um die Abbildungseigenschaften zu optimieren, und die Hornhaut besitzt nicht mehr die natürliche Form, von der die Berechnungsformeln ausgehen.

Wünschenswert ist daher, die erzielte Refraktion noch während der Operation, nachdem die Intraokularlinse eingesetzt wurde, überprüfen zu können, um die korrekte Refraktion zu bestätigen oder gegebenenfalls sofort Korrekturmaßnahmen zu ergreifen. Einige Chirurgen messen dazu die Refraktion nach Implantation der Intraokularlinse mit einem tragbaren Autorefraktor. Dies ist allerdings umständlich: Die Operation muss unterbrochen werden, um das Operationsmikroskop zur Seite zu schwenken, damit der Autorefraktor vom Chirurgen oder einem Assistenten über den liegenden Patienten gehalten werden kann. Die erforderlichen Maßnahmen zur Sicherung der Sterilität verzögern den Ablauf zusätzlich.

Der Erfindung liegt die Aufgabe zu Grunde, ein Operationsmikroskop vorzustellen, das eine leichte Überprüfung der Refraktion des Patientenauges ermöglicht. Ausgehend vom eingangs genannten Stand der Technik wird die Aufgabe gelöst durch die Merkmale des unabhängigen Anspruchs. Vorteilhafte Ausführungsformen finden sich in den Unteransprüchen.

Erfindungsgemäß umfasst das Operationsmikroskop eine Abbildeanordnung und eine Brechungsanordnung. Die Abbildeanordnung kann einen Aktivzustand und einen Passivzustand einnehmen. Im Aktivzustand erzeugt die Abbildeanordnung eine Strukturabbildung, die den Beobachtungsstrahlengang überlagert. Im Passivzustand der Abbildeanordnung wird der Beobachtungsstrahlengang nicht durch die Abbildeanordnung beeinflusst. Die Brechungsanordnung kann ebenfalls einen Aktivzustand und einen Passivzustand einnehmen. Im Aktivzustand weitet die Brechungsanordnung den Beobachtungsstrahlengang auf. Im Passivzustand ist der Beobachtungsstrahlengang nicht durch die Brechungsanordnung beeinflusst.

Zunächst werden einige Begriffe erläutert. In dem Operationsmikroskop wird mit der Frontlinse, dem Vergrößerungssystemen und dem Einblick ein Beobachtungsstrahlengang definiert. Der Beobachtungsstrahlengang kann in einer Arbeitsebene fokussiert werden, die der Anschaulichkeit halber als Augenlinsenebene bezeichnet wird. Für die stereoskopische Betrachtung des Operationsfeldes definiert das Operationsmikroskop normalerweise zwei Beobachtungsstrahlengänge. Der Einblick für den Beobachter hat dann zwei Okulare. Es können mehrere Einblicke für mehrere Beobachter vorgesehen sein.

Eine Strukturabbildung umfasst kleinteilige Strukturen, so dass leicht erkennbar ist, ob die Abbildung scharf ist oder nicht. Insbesondere soll dies gelten, wenn die Strukturabbildung auf der Retina des Patienten abgebildet ist. Eine Strukturabbildung oder ein Strahlengang liegen dann in Form paralleler Strahlen vor, wenn sie auf unendlich abgebildet sind. Es kann ein teildurchlässiger Spiegel vorgesehen sein, mit dem die Strukturabbildung dem Beobachtungsstrahlengang überlagert werden kann.

Wenn der Augenchirurg die Kataraktoperation durchführt, arbeitet er in der Ebene der Augenlinse. Die Abbildeanordnung sowie die Brechungsanordnung sind während der Operation im Passivzustand und das Operationsmikroskop ist in der Augenlinsenebene fokussiert. Wenn die Intraokularlinse eingesetzt wurde, soll der Augenchirurg intraoperativ prüfen, ob die Intraokularlinse passt. Bei dem erfindungsgemäßen Operationsmikroskop werden zu diesem Zweck die Abbildeanordnung und die Brechungsanordnung in den Aktivzustand gebracht. Mit der Abbildeanordnung wird eine Strukturabbildung erzeugt, die auf die Intraokularlinse auftrifft und die entsprechend den optischen Eigenschaften der Intraokularlinse auf der Retina abgebildet wird. Der Strahlengang der Strukturabbildung trifft vorzugsweise in der gleichen Richtung auf der Intraokularlinse auf wie der Beobachtungsstrahlengang, weiter vorzugsweise sind die beiden Strahlengänge koaxial bezogen auf ihre jeweiligen Zentrumsstrahlen. Mit der ebenfalls im Aktivzustand befindlichen Brechungsanordnung wird dem Augenchirurg die Möglichkeit gegeben, die Strukturabbildung auf der Retina zu betrachten, ohne dass eine darüber hinausgehende Anpassung des Beobachtungsstrahlengangs erforderlich wäre. Indem der Beobachtungsstrahlengang aufgeweitet wird, ist er nicht mehr in der Augenlinsenebene fokussiert, sondern trifft als paralleles, als leicht konvergentes oder als leicht divergentes Strahlenbündel in der Augenlinsenebene auf. Mit dem aufgeweiteten Beobachtungsstrahlengang blickt der Chirurg durch die Intraokularlinse hindurch und sieht die Strukturabbildung auf der Retina. Führt die Überprüfung zu dem Ergebnis, dass die neue Linse passt, können die Abbildungsanordnung und die Brechungsanordnung wieder in den Passivzustand gebracht werden. Das Operationsmikroskop ist dann sofort wieder in der Augenlinsenebene fokussiert, so dass die Operation ohne Verzögerung fortgesetzt werden kann.

Diese Überprüfung der Refraktion ist für den Augenchirurg sehr komfortabel. Das Operationsmikroskop kann während der Überprüfung die Position und die Einstellung beibehalten, die es für die eigentliche Operation haben muss. Für die Überprüfung werden lediglich Veränderungen an der Brechungsanordnung und der Abbildeanordnung gemacht, die gezielt für die Überprüfung eingerichtet sind. Die Brechungsanordnung ist vorzugsweise zwischen der Frontlinse und der Augenlinsenebene angeordnet. Der Strahlengang der Strukturabbildung wird vorzugsweise zwischen der Frontlinse und der Augenlinsenebene dem Beobachtungsstrahlengang überlagert.

Wie der Strahlengang der Strukturabbildung genau aussehen muss (parallel, leicht konvergent oder leicht divergent), damit auf der Retina eine scharfe Strukturabbildung entsteht, hängt von den optischen Eigenschaften der Intraokularlinse ab. Im Idealfall ist die Intraokularlinse, die in entspanntem Zustand eingesetzt wird, auf unendlich adaptiert. Eine scharfe Abbildung auf der Retina entsteht in diesem Fall, wenn die Strukturabbildung in Form eines parallelen Strahlenbündels auf die Intraokularlinse auftrifft. Damit der unproblematische Fall einer auf unendlich adaptierten Intraokularlinse schnell und unkompliziert überprüft werden kann, kann für die Abbildeanordnung ein Modus vorgesehen sein, in dem die Strukturabbildung in Form paralleler Strahlen auf die Augenlinsenebene trifft.

Damit der Chirurg in diesem Fall die Abbildung auf der Retina scharf sehen kann, muss der Beobachtungsstrahlengang ebenfalls in Form paralleler Strahlen auf die Augenlinsenebene auftreffen. Für die Brechungsanordnung ist deswegen vorzugsweise ein Modus vorgesehen, mit dem ein in der Augenlinsenebene fokussierter Beobachtungsstrahlengang in einen parallel in der Augenlinsenebene auftreffenden Beobachtungsstrahlengang umgewandelt wird, wenn die Brechungsanordnung vom Passivzustand in den Aktivzustand wechselt. Die Brechungsanordnung kann beispielsweise eine Zerstreuungslinse sein, die in den optischen Strahlengang des Mikroskops eingebracht wird, so dass der virtuelle Brennpunkt der Zerstreuungslinse mit dem Brennpunkt der Frontlinse zusammenfällt.

Wenn die Intraokularlinse nicht auf unendlich adaptiert ist, müssen der Strahlengang der Strukturabbildung und der Beobachtungsstrahlengang leicht konvergent bzw. leicht divergent sein. Der Grad der Konvergenz bzw. Divergenz muss in dem Strahlengang der Strukturabbildung und im Beobachtungsstrahlengang übereinstimmen, damit sowohl eine scharfe Strukturabbildung auf der Retina entsteht als auch der Chirurg diese scharf betrachten kann. Das Operationsmikroskop umfasst deswegen vorzugsweise einen Modus, in dem der Beobachtungsstrahlengang und der Strahlengang der Strukturabbildung den gleichen Grad an Konvergenz bzw. Divergenz haben, wenn die Abbildungsanordnung und die Brechungsanordnung vom Passivzustand in den Aktivzustand wechseln.

Das Operationsmikroskop kann so eingerichtet sein, dass die Brechungsanordnung und die Abbildeanordnung beim Wechsel zwischen dem Passivzustand und dem Aktivzustand separat bedient werden. Komfortabler für den Augenchirurg ist es, wenn eine gemeinsame Bedienung der Abbildeanordnung und der Brechungsanordnung möglich ist. Die Abbildeanordnung und die Brechungsanordnung können zu diesem Zweck in einem Zusatzmodul des Operationsmikroskops zusammengefasst sein, das. Mit dem Zusatzmodul können die Abbildeanordnung und die Brechungsanordnung gemeinsam zwischen dem Aktivzustand und dem Passivzustand wechseln. Für den Wechsel zwischen Aktivzustand und Passivzustand kann das Modul beispielsweise schwenkbar mit dem Operationsmikroskop verbunden sein.

Wenn das Zusatzmodul um die optische Achse der Frontlinse drehbar ist, kann die Untersuchung der Intraokularlinse in verschiedenen Meridianen durchgeführt werden, so dass auch ein Astigmatismus erkannt werden kann.

Zum Erzeugen der Strukturabbildung kann in der Abbildeanordnung ein Struktursubstrat vorgesehen sein. Wenn Licht durch das Struktursubstrat hindurchtritt, entsteht ein der Struktur entsprechendes Strahlenbündel, mit dem auf einer Fläche eine Abbildung der Struktur erzeugt werden kann. Das Struktursubstrat ist vorzugsweise ein Mikrostruktursubstrat, das wesentlich kleiner ist als die Strukturabbildung. In der Abbildeanordnung kann ein Objektiv vorgesehen sein, mit dem das von dem Struktursubstrat ausgehende Strahlenbündel in ein paralleles Strahlenbündel umgewandelt wird. Die Strukturabbildung wird damit auf unendlich abgebildet. Anschließend werden die Lichtstrahlen vorzugsweise so umgelenkt, dass sie parallel zum Beobachtungsstrahlengang in der Augenlinsenebene auftreffen.

Zum Erzeugen der Strukturabbildung kann das Operationsmikroskop eine separate Lichtquelle haben, die in dem Zusatzmodul enthalten sein kann. Es kann auch das Struktursubstrat eine selbstleuchtende Struktur aufweisen, z.B. in Form eines Mikrodisplays. In einer vorteilhaften Ausführungsform wird zum Erzeugen der Strukturabbildung eine Lichtquelle verwendet werden, die in dem Operationsmikroskop ohnehin als Mikroskopbeleuchtung vorhanden ist. Der Beleuchtungsstrahlengang der Mikroskopbeleuchtung erstreckt sich regelmäßig durch dieselbe Frontlinse, durch die auch der Beobachtungsstrahlengang hindurch geht. Die Abbildeanordnung kann so eingerichtet sein, dass sie im Aktivzustand im Beleuchtungsstrahlengang der Mikroskopbeleuchtung angeordnet ist. Wenn die Abbildeanordnung im Passivzustand ist, beleuchtet die Mikroskopbeleuchtung direkt die Augenlinse. Zum Erzeugen der Strukturabbildung ist es häufig besser, wenn das Licht nicht diesen direkten Weg nimmt. Am Eingang der Abbildeanordnung kann ein Prisma vorgesehen sein, um den Beleuchtungsstrahlengang in eine geeignete Richtung abzulenken.

Mit dem einfachen Wechsel zwischen Aktivzustand und Passivzustand kann man mit dem erfindungsgemäßen Operationsmikroskop leicht feststellen, ob die frisch eingesetzte Intraokularlinse die richtige Refraktion hat oder nicht. Wenn der Augenchirurg durch das Operationsmikroskop hindurch eine scharfe Strukturabbildung auf der Retina sieht, ist alles in Ordnung und die Operation kann abgeschlossen werden. Sieht der Augenchirurg eine unscharfe Strukturabbildung, so passt die Intraokularlinse nicht. Allerdings ist der unscharfen Strukturabbildung nicht direkt zu entnehmen, wie stark die Intraokularlinse von den passenden Werten abweicht.

Das Operationsmikroskop ist vorzugsweise so eingerichtet, dass zusätzlich für den Augenchirurg leicht die Information zugänglich wird, wie weit die nicht passende Intraokularlinse von den richtigen Werten abweicht. Es gibt mehrere Möglichkeiten, wie diese Information bereitgestellt werden kann. Nach einem Ansatz ist die Abbildeanordnung so einstellbar, dass trotz der nicht passenden Intraokularlinse eine scharfe Strukturabbildung auf der Retina entsteht. Dies kann erreicht werden, indem in der Abbildeanordnung die Länge des Lichtwegs zwischen dem Struktursubstrat und dem Objektiv verändert wird. Die Strukturabbildung trifft dann nicht mehr als paralleles, sondern als leicht konvergentes oder divergentes Strahlenbündel auf die Intraokularlinse auf. Je größer die Abweichung vom parallelen Strahlenbündel sein muss, um eine scharfe Strukturabbildung auf der Retina zu erzeugen, desto stärker weicht die eingesetzte Intraokularlinse von den richtigen Werten ab. Um die Länge des Lichtwegs zu verändern, kann der Abstand zwischen dem Objektiv und dem Struktursubstrat verändert werden. Das Objektiv und/oder das Struktursubstrat können so gestaltet sein, dass sie in der entsprechenden Richtung bewegt werden können. Vorzugsweise ist eine Skala vorgesehen, auf der abgelesen werden kann, um wieviel die Länge des Lichtwegs verändert wurde.

Der Verstellweg in der Abbildeanordnung ist vorzugsweise so gewählt, dass er den Bereich von plus drei Dioptrien bis minus drei Dioptrien, vorzugsweise den Bereich von plus fünf Dioptrien bis minus fünf Dioptrien umfasst. Wenn sich mit diesem Verstellbereich keine scharfe Abbildung erzeugen lässt, ist die Linse in jedem Fall so unbrauchbar, dass sie ersetzt werden muss. Andererseits sollte der Verstellweg auch nicht zu groß gewählt werden, weil die richtige Einstellung dann zu viel Aufwand erfordert. Der Verstellweg kann deswegen so begrenzt sein, dass er sich innerhalb des Bereichs von plus 20 Dioptrien bis minus 20 Dioptrien, vorzugsweise innerhalb des Bereichs von plus 10 Dioptrien bis minus 10 Dioptrien bewegt. Für den Verstellweg der Brechungsanordnung können die entsprechenden Bereiche gelten.

Alternativ können die unterschiedlichen Längen des Lichtwegs auch dadurch eingestellt werden, dass mehrere Struktursubstrate und/oder Objektive vorgesehen sind, die in jeweils festem Abstand zueinander stehen und die wahlweise in den Strahlengang eingebracht bzw. aus diesem entfernt werden können. Nach wiederum einer anderen Möglichkeit kann die Abbildeanordnung eine Mehrzahl von Struktursubstraten aufweisen, die einen unterschiedlichen Abstand zum Objektiv haben und die gleichzeitig in den Strahlengang eingebracht werden. Wenn die Abbildungen der Struktursubstrate sich nicht überdecken, kann der Augenchirurg sie gleichzeitig beobachten und feststellen, welche der Abbildungen scharf auf der Retina abgebildet wird.

Nach einem anderen Ansatz kann die Abbildeanordnung so eingerichtet sein, dass mehrere Strukturabbildungen parallel zueinander auf die Intraokularlinse treffen. Wenn die Intraokularlinse passt, vereinigen sich die Strukturabbildungen wieder und werden auf der Retina scharf abgebildet. Passt die Intraokularlinse nicht, sind die Strukturabbildungen auf der Retina zueinander versetzt. Es kann ein Farbfilter vorgesehen sein, um die Strukturabbildungen voneinander zu unterscheiden.

Die zueinander parallelen Strukturabbildungen können dadurch erzeugt werden, dass ein von dem Struktursubstrat ausgehendes Strahlenbündel in mehrere Teilbündel unterteilt wird. Dazu kann hinter dem Objektiv ein optisches Modul angeordnet sein, mit dem das durch das Objektiv tretende Strahlenbündel in mehrere Teilbündel unterteilt werden kann. Das optische Modul kann eine Blende mit mehreren Apperturen sein. Bei passender Intraokularlinse vereinigen sich die Teilbündel auf der Retina zu einer einzigen deckungsgleichen Strukturabbildung. Bei nicht passender Intraokularlinse bilden die Teilbündel mehrere nebeneinander liegende Strukturabbildungen. Die Strukturabbildungen können mit einer Skala versehen sein, so dass der Abstand direkt ablesbar ist.

Alternativ können die parallelen Strukturabbildungen auch mit mehreren Struktursubstraten erzeugt werden. Für jedes der Struktursubstrate kann ein Objektiv vorgesehen sein, so dass die Strahlenbündel parallel und parallel zueinander auf die Intraokularlinse auftreffen. Es kann jeweils ein gleich langer Lichtweg zwischen dem Struktursubstrat und dem zugehörigen Objektiv liegen. Es können gemeinsame Sammellinsen oder Zerstreuungslinsen vorgesehen sein, so dass die Fokussierung der Strahlenbündel gemeinsam geändert werden kann.

Wenn die Intraokularlinse nicht passt und die Strukturabbildungen deswegen unscharf auf der Retina erscheint, so hat dies zugleich zur Folge, dass der parallel von der Augenlinsenebene ausgehende Beobachtungsstrahlengang ebenfalls nicht richtig auf die Retina fokussiert ist. Die Brechungsanordnung kann so gestaltet sein, dass dies ausgeglichen werden kann, so dass trotzdem ein scharfes Bild der Retina für den Augenchirurgen erzeugt werden kann.

Neben diesen Ausführungsbeispielen sind weitere möglich. Alle Linsen können auch als neuartige Linsen mit veränderbarer Brennweite ausgeführt sein, bei denen durch Anlegen einer Spannung die Oberflächenkrümmung und damit die Brennweite einer linsenförmig angeordneten Flüssigkeit oder eines Polymers geändert werden. Alle Verschiebungen, Drehungen können elektromotorisch erfolgen. Als Mikrostrukturen können auch selbstleuchtende Strukturen eingesetzt werden, z.B. Mikrodisplays. Die Aufteilung eines Strahlenbündels in Teilbündel kann auch durch pupillenteilende Prismenanordnungen oder diffraktive optische Elemente erfolgen.

Allen Ausführungsformen ist gemeinsam, dass mit einem oder mehreren Beleuchtungsstrahlengängen Mikrostrukturen auf die Retina des Patientenauges abgebildet werden. Diese Mikrostrukturen werden durch eine Zusatzoptik für den Chirurgen im Mikroskop beobachtbar und aus ihrer Schärfe, Form und Lage zueinander kann die Refraktion des Patientenauges mit implantierter Intraokularlinse bestimmt werden. Selbstverständlich kann die Beobachtung der Strukturen auch über ein Kamerasystem erfolgen, und mit elektronischer Bildauswertung kann die Refraktion auch automatisch bestimmt werden.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine schematische Frontansicht eines Operationsmikroskops;
- Fig. 2:: das Operationsmikroskop aus Fig. 1 in Seitenansicht;
- Fig.3a,3b:: ein erstes Ausführungsbeispiel;
- Fig. 4:: ein erfindungsgemäßes Zusatzmodul in einer anderen Ausführungsform;
- Fig. 5a,5b:: ein Operationsmikroskop mit einer Brechungsanordnung;
- Fig. 6a,6b:: die Abbildung eines Siemenssterns auf die Retina bei der Ausführungsform gemäß Fig. 3a, wobei der Übersichtlichkeit halber die Umlenkung des Strahlengangs nicht dargestellt ist;
- Fig. 7:: ein zweites Ausführungsbeispiel;

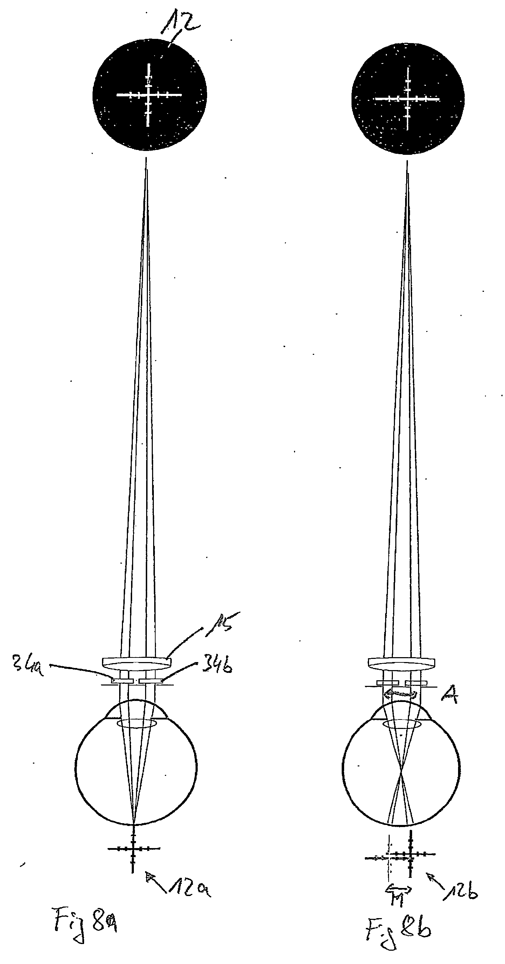

- Fig. 8a,8b:: die Abbildung eines Fadenkreuzes mit zwei Teilbündeln auf die Retina im zweiten Ausführungsbeispiel, wobei zur Verdeutlichung die Umlenkung des Strahlengangs nicht dargestellt ist;
- Fig. 9:: ein drittes Ausführungsbeispiel;
- Fig. 10a,10b:: die Abbildung der zwei sich ergänzenden Mikrostrukturen auf die Retina im dritten Ausführungsbeispiel, wobei der Übersichtlichkeit halber die Umlenkung des Strahlengangs nicht dargestellt ist;
- Fig. 11:: schematisch in einem Bild zusammengefasst die verschiedenen Einstellmöglichkeiten der Linsen und der Mikrostrukturplatte;
- Fig. 12:: ein viertes Ausführungsbeispiel;

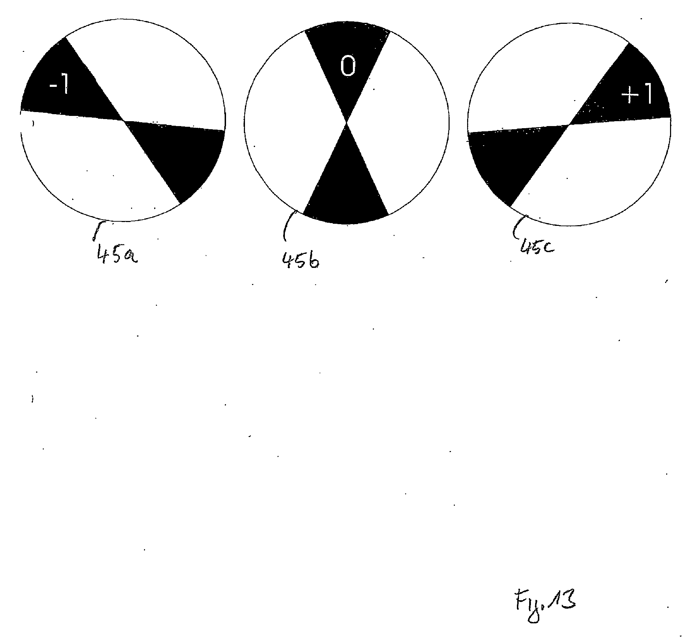

- Fig. 13:: dem vierten Beispiel zugeordnete Mikrostrukturen;
- Fig. 14a-14c:: die Abbildungen der Mikrostrukturen auf die Retina im vierten Ausführungsbeispiel, wobei der Übersichtlichkeit halber die Umlenkung des Strahlengangs nicht dargestellt ist.

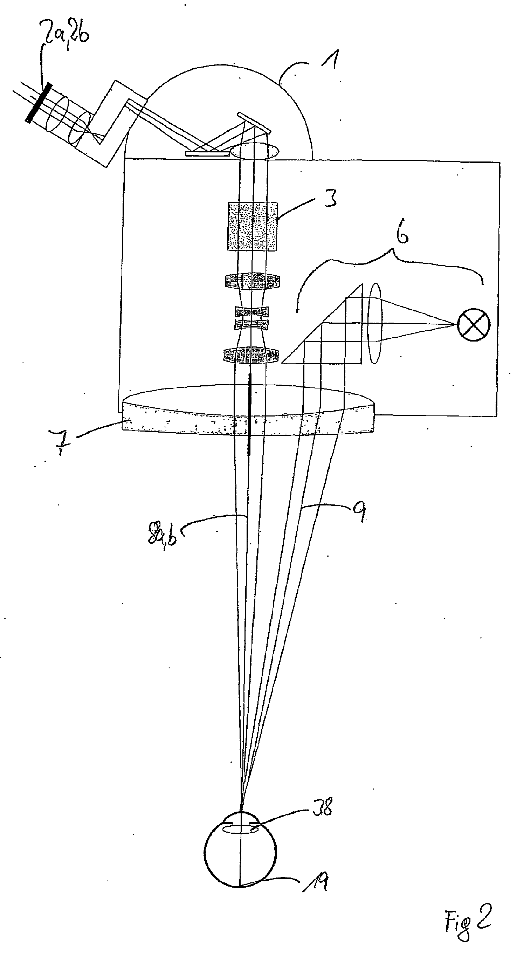

Vom Beobachter aus betrachtet sind die typischen Komponenten eines Operationsmikroskops Fig.l, Fig. 2:
- ein Einblick 1 mit zwei Okularen 2a, 2b für einen ersten Beobachter und gegebenenfalls weitere optische Strahlengänge mit weiteren Einblicken
- Strahlenteiler 3 zum Auskoppeln eines Teiles des optischen Strahlengangs und Umlenkung, z.B. auf eine Videokamera 42 oder andere Zubehörkomponenten
- ein Vergrößerungssystem 4 mit einstellbarem Vergrößerungsfaktor, z.B. ein Galilei Vergrößerungswechsler oder ein Zoom, zur vergrößerten Betrachtung des Patientenauges 5 oder eines anderen Objekts.
- Ein Beleuchtungssystem 6 zur Beleuchtung des Patientenauges 5 durch eine gemeinsame Frontlinse 7 für alle Beobachtungsstrahlengänge 8a,8b und mindestens einen Beleuchtungsstrahlengang 9.
- Im Bereich zwischen der Frontlinse 7 und dem Patientenauge 5 optional weiteres Zubehör, z.B. Vorsatzoptiken zur Beobachtung des Augenhintergrunds.

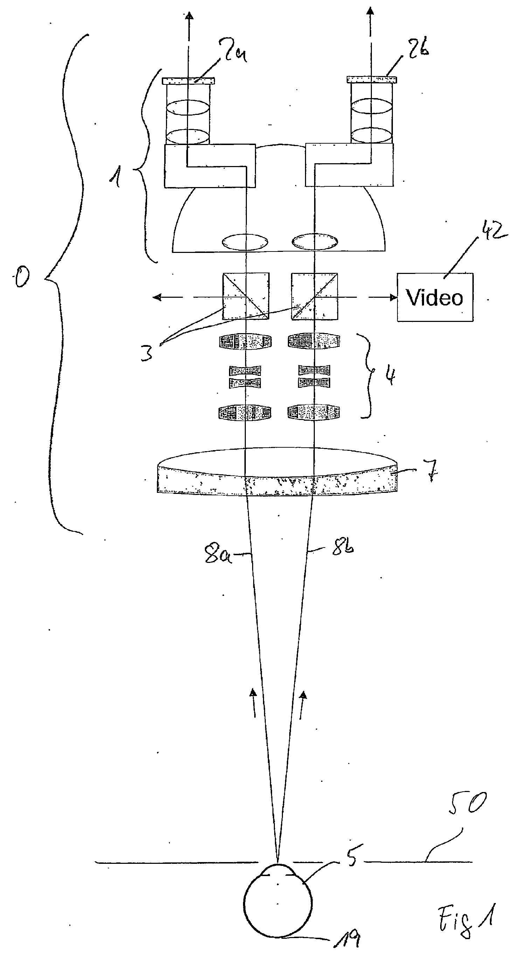

Beim Blick durch die rechten und linken Okulare der Einblicke stellt sich dem Beobachter ein stereoskopisches, vergrößertes Bild des Objekts, z.B. von dem operierten Patientenauges, dar. Das Operationsmikroskop gemäß den Figuren 1 und 2 ist dazu geeignet, mit einem erfindungsgemäßen Zusatzmodul ausgerüstet zu werden.

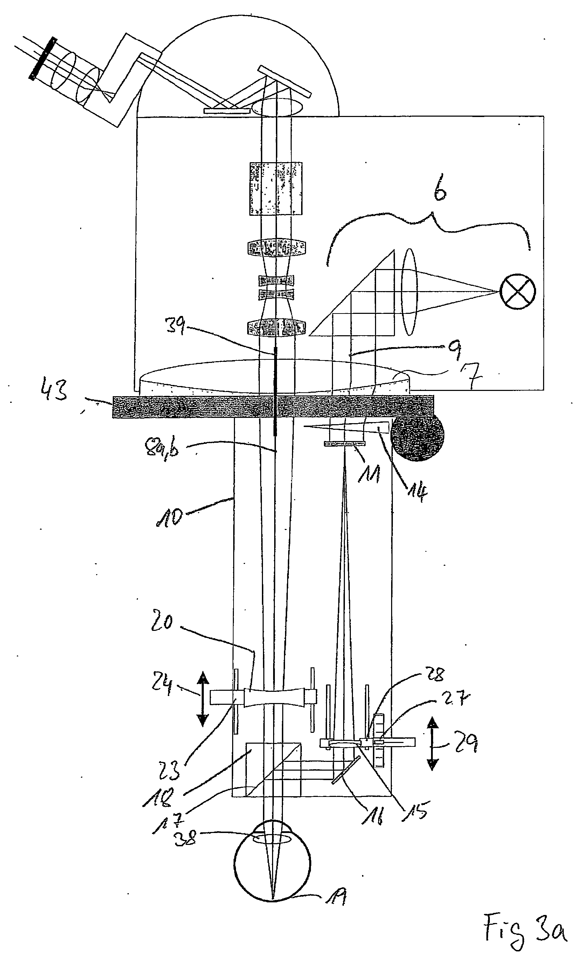

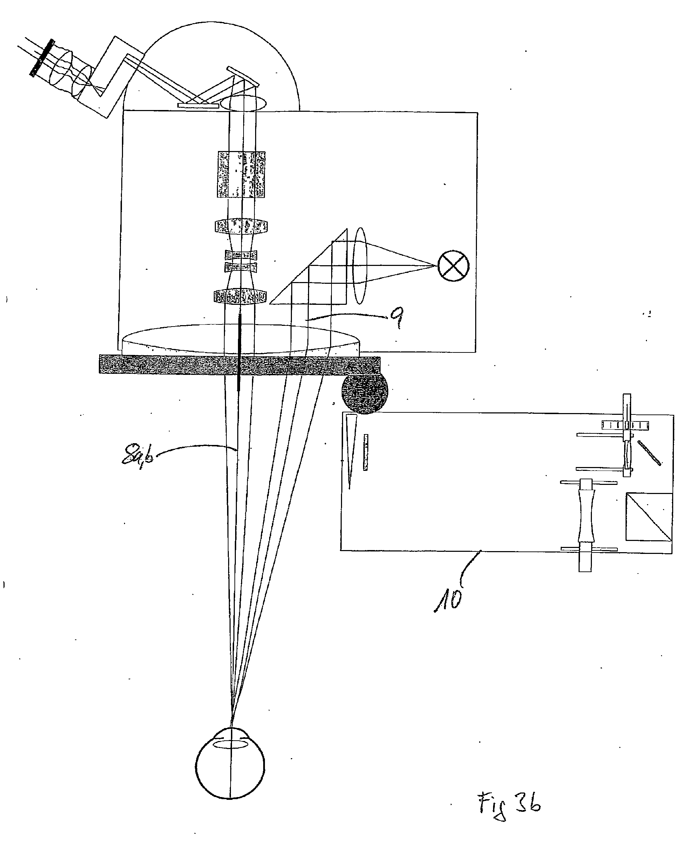

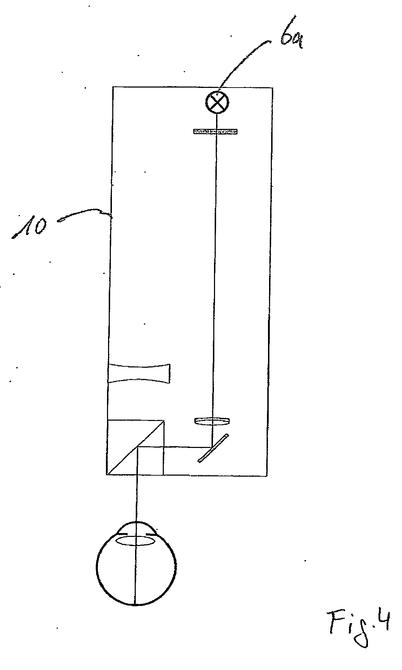

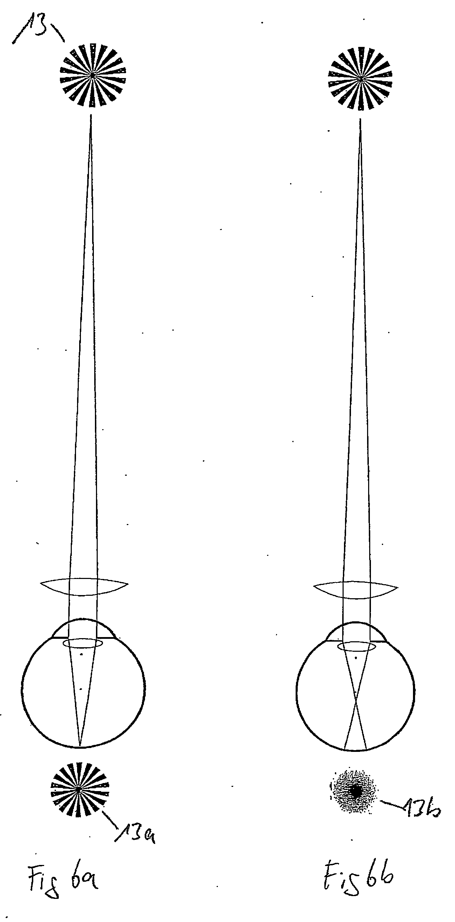

Im ersten Ausführungsbeispiel (Fig. 3a, 3b) verfügt das Zusatzmodul 10 über eine Mikrostrukturplatte 11, die von der Mikroskopbeleuchtung 6 hinterleuchtet wird. Unter Mikrostrukturplatte 11 soll hier ein optisches Substrat mit beliebigen geometrischen Strukturen/Objekten zum Abbilden durch ein Objektiv gemeint sein, z.B. ein als Siemensstern 13 bekanntes Speichenmuster (Fig. 6a,b). Die Mikrostrukturplatte 11 ist ein Struktursubstrat im Sinne der Erfindung. Dazu wird vorteilhafterweise der Beleuchtungsstrahlengang 9 durch ein Prisma 14 geringfügig abgelenkt, um die Mikrostrukturplatte 11 gut auszuleuchten. Prinzipiell kann die Hinterleuchtung aber auch durch eine separate Lichtquelle 6a geschehen (Fig. 4), z.B. durch LED oder Halogenlichtquellen, die in das Zusatzmodul 10 integriert sein können.

Die Mikrostrukturplatte 11 wird von einem Objektiv 15 nach unendlich abgebildet. Der weitere Strahlengang verläuft über einen Umlenkspiegel 16 und einen teildurchlässigen Spiegel 17, der als Strahlenteilerwürfel 18 ausgeformt ist. Dieser lenkt einen Teil z.B. 50% des von der Mikrostrukturplatte 11 ausgehenden Lichts in das Patientenauge 5. Bei auf die Ferne eingestelltem Auge wird ein scharfes Bild der Mikrostrukturplatte 11 auf der Retina 19 erzeugt. Das Prisma 14, die Mikrostrukturplatte 11, das Objektiv 15, der Umlenkspiegel 16 und der teildurchlässige Spiegel 17 bilden gemeinsam eine erfindungsgemäße Abbildeanordnung. In Fig. 3a ist das Zusatzmodul 10 mit der Abbildeanordnung 11, 14, 15, 16, 17 und der Brechungsanordnung 20 im Aktivzustand. In Fig. 3b ist das Zusatzmodul im Passivzustand.

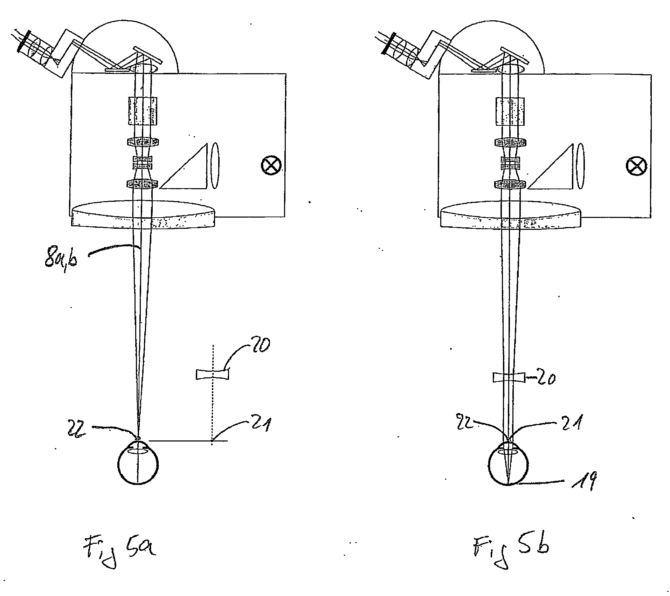

Eine weitere Optik 20 (Fig. 3a), z.B. eine Streulinse des Moduls passt den Strahlengang 8a, 8b des Mikroskops an den des Patientenauges 5 an, sodass bei eingeschaltetem Modul 10 der Chirurg die Retina 19 des Patientenauges 5 scharf abgebildet in den Okularen 2a, 2b wahrnimmt. Dazu wird die Streulinse 20 in den Strahlengang 8a, 8b des Operationsmikroskops eingebracht, sodass der Brennpunkte 21 dieser Linse mit dem Brennpunkt 22 der Frontlinse zusammenfällt Fig. 5a, 5b. Die Linse 20 ist entweder fix oder auf einem Schlitten 23 beweglich, um durch Hoch- und Herunterschieben 24 eine Fokussierung der Strahlengänge 8a, 8b auf die Retina 19 vorzunehmen, wenn das Auge nicht auf unendlich eingestellt sein sollte. Die Optik 20 ist eine erfindungsgemäße Brechungsanordnung.

Der Chirurg kann nun die abgebildete Mikrostruktur 13a auf der Retina 19 durch den Strahlenteiler 17 hindurch beobachten (Fig 3a). Der Verlauf der Abbildungsstrahlengänge ist in Fig. 6a und Fig. 6b, der Übersichtlichkeit halber unter Auslassung der Umlenkungen, dargestellt. Wenn das Patientenauge auf die Ferne unendlich korrigiert ist, beobachtet der Chirurg in den Okularen 2a, 2b ein scharfes Bild 13a der Mikrostruktur 13 auf der Retina 19. Ist das Patientenauge nach Implantation der Intraokularlinse nicht auf die Ferne korrigiert, wird die Mikrostruktur 13 unscharf abgebildet 13b. Aus dem Grad der Unschärfe, z.B. bei der Abbildung eines Siemenssterns 13, kann auf den Refraktionsfehler geschlossen werden. In den Fällen, in denen eine Fehlsichtigkeit erkannt wird, kann auch auf einen separaten Autorefraktor zurückgegriffen werden, um für Korrekturmaßnahmen den genauen Refraktionszustand zu messen.

Das Objektiv 15 kann auf einem Linearschlitten 28 verschoben werden, um die Mikrostruktur scharf auf die Retina abzubilden. An einer kalibrierten Skala 27 kann die dazu erforderliche Verschiebung der Linse bestimmt und daraus der Refraktionsfehler berechnet werden. Alternativ kann auch die Mikrostrukturplatte 11 auf einem Schlitten 25 aus Ihrer Grundstellung zum Objektiv 15 hin oder weg 26 verschoben werden, um die Mikrostrukturplatte 11 auf die Retina 19 scharf abzubilden. An der Skala 27a kann dann die Verschiebung abgelesen und der Refraktionsfehler bestimmt werden. Alternativ kann selbstverständlich auch ein geeignetes Korrekturglas 30 zwischen dem Strahlenteilerwürfel 18 und dem Objektiv 15 eingesetzt werden, um eine scharfe Abbildung auf die Retina einzustellen. Aus der optischen Stärke des dafür notwendigen Korrekturglases 30 kann dann der Refraktionsfehler bestimmt werden.

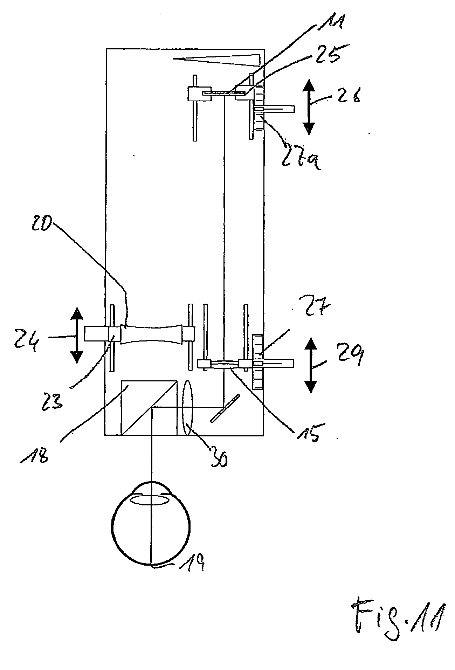

Die genannten verschiedenen Einstellmöglichkeiten der Linsen 15, 20 und der Mikrostrukturplatte 11 sind in Fig. 11 zusammengefasst. An der Skala 27 kann die Verschiebung der Linse 15 und an der Skala 27a die Verschiebung der Mikrostrukturplatte abgelesen werden. Die Linse 20 wird zur Scharfstellung des Mikroskopbildes verschoben.

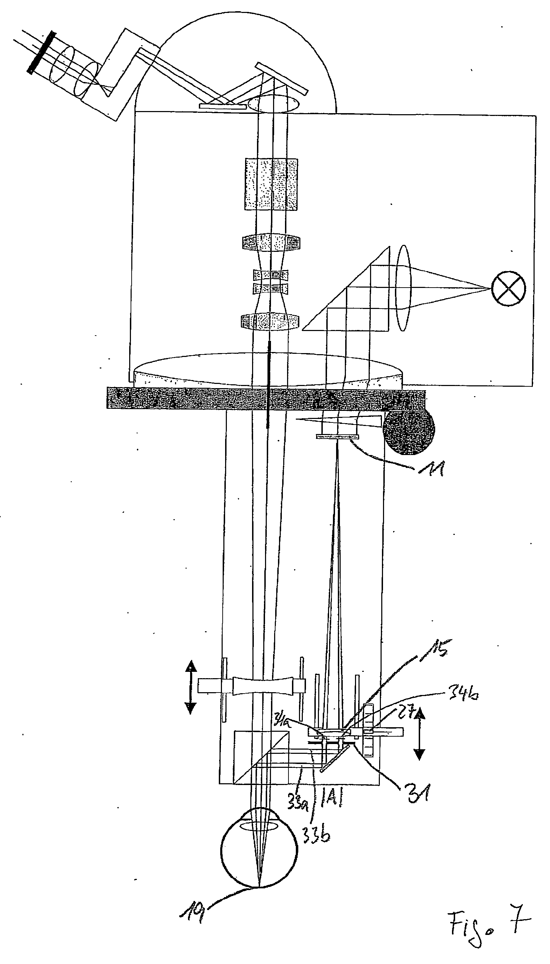

In einem zweiten Ausführungsbeispiel gemäß Fig. 7 wird ebenfalls eine hinterleuchtete Mikrostrukturplatte 11 von einem Objektiv 15 nach unendlich abgebildet. Die Mikrostruktur kann ein Fadenkreuz mit Hilfslinien 12 sein. Zusätzlich ist jetzt eine Blende 31 mit zwei Aperturen 32a, 32b zwischen dem Objektiv 15 und dem Strahlenteilerwürfel 18 angebracht, sodass zwei gegeneinander um den Abstand A der Aperturen versetzte parallele Teilbündel 33a, 33b entstehen. Diese Teilbündel sind selbstverständlich weiterhin kollimiert und vereinigen sich bei einem auf die Ferne korrigierten Auge nach wie vor auf der Retina 19, wenn das Patientenauge auf unendlich eingestellt ist und erzeugen gemeinsam ein scharfes Bild des Fadenkreuzes 12a.

Weicht die Refraktion von unendlich ab (12b), werden die Teilbündel 33a, 33b auf der Retina zu gegeneinander verschobenen Bildern der Mikrostruktur 12 gebündelt und sind zusätzlich unscharf. Der Chirurg kann nun den Grad des bestehenden Refraktionsfehlers aus der Verschiebung der Teilbilder auf der Retina erkennen, was leichter fällt als die Bewertung der Abbildungsschärfe und was außerdem Ablesefehler durch Akkommodation des Chirurgen ausschließt. Der Mittenabstand M der beiden Teilbilder auf der Retina hängt mit der Brennweite des Auges f gemäß M=A*b-f/f, (A: Abstand der Teilbündel, b: Bildweite) zusammen. Der Chirurg kann Anhand der Hilfslinien den Mittenabstand M erkennen.

Das Zusatzmodul 10 kann mit einem Drehlager 43 um die optische Achse 39 der Frontlinse gedreht werden. Dadurch kann die Refraktion des Auges in unterschiedlichen Meridianen bestimmt werden, sodass auch ein bestehender Astigmatismus erkennbar ist.

Es können natürlich auch mehr als zwei Teilbündel durch mehrere Aperturen erzeugt werden, z.B. drei oder vier Aperturen, um ebenfalls einen Astigmatismus zu erkennen. Dieser äußert sich dann dadurch, dass die Teilbilder der Mikrostruktur in vertikaler Richtung einen anderen Abstand voneinander besitzen als die Kreuze in horizontaler Richtung.

Um die von den Teilbündeln erzeugten Abbildungen gut zu unterscheiden, können in die Strahlengänge jeweils unterschiedliche Farbfilter 34a, 34b eingesetzt werden. Dadurch ist auch erkennbar, ob die Refraktion des Auges zu stark oder zu schwach ist, je nachdem, auf welcher Seite die farblich gekennzeichneten Bilder des Fadenkreuzes 12 zueinander liegen.

Wie an dem ersten Ausführungsbeispiel erläutert können auch hier alternativ die Mikrostrukturplatte 11 oder das Objektiv 15 verschoben werden, um die zueinander versetzten Bilder des Fadenkreuzes auf der Retina zur Deckung zu bringen. Die erforderliche Verschiebung, die jeweils an den Skalen 27 oder 27a abgelesen werden kann, ergibt die Abweichung der Refraktion von unendlich. Wie im vorigen Beispiel beschrieben kann auch ein Kompensationsglas 30 verwendet werden, um die bestehende Fehlsichtigkeit zu ermitteln.

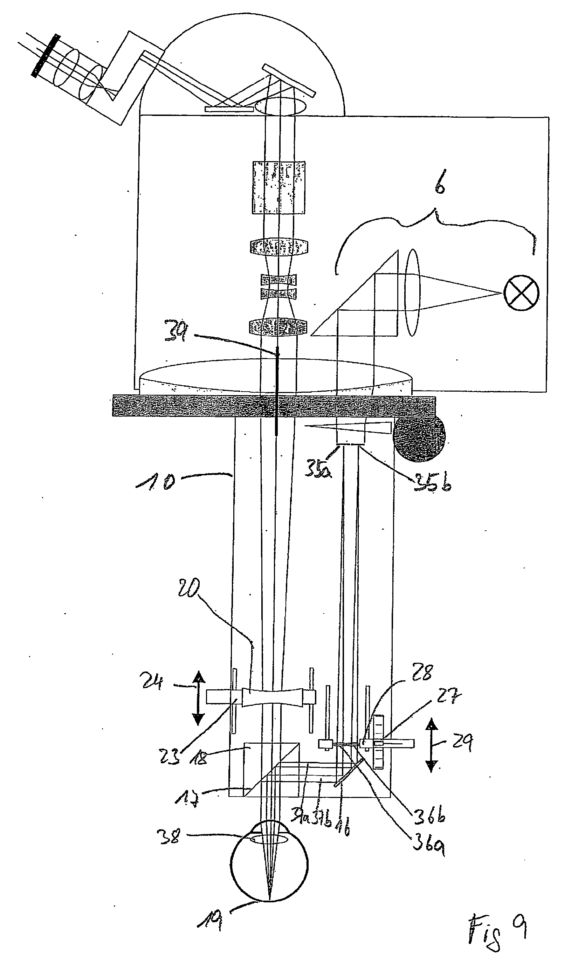

In einem dritten Ausführungsbeispiel gemäß Fig. 9 verfügt das Modul 10 über zwei Mikrostrukturplatten 35a, 35b, die von der Mikroskopbeleuchtung 6 hinterleuchtet werden. Die Mikrostrukturen, beispielsweise ein Doppel-T 40 und ein Doppelbalken 41 sind so ausgeformt, dass sich ihre Bilder ergänzen und auch eine geringe Verschiebung der Bilder zueinander gut erkannt werden kann.

Die beiden Mikrostrukturplatten sind gegeneinander seitlich um einen Abstand A versetzt. Jeder dieser Mikrostrukturplatten ist ein separates Objektiv 36a, 36b zugeordnet, dass die jeweilige Mikrostrukturplatte 35a, 35b nach unendlich abbildet. Die Objektive sind ebenfalls um den Abstand A seitlich gegeneinander versetzt und so angeordnet, dass zwei zueinander parallele Strahlengänge 37a, 37b, deren optische Achsen um den Abstand A gegeneinander verschoben sind, entstehen. Diese Strahlengänge werden wie bereits beschrieben durch Spiegel 16,17,18 in das Patientenauge gelenkt.

Der Abstand A darf nicht zu groß gewählt werden, damit noch beide Strahlengänge 37a, 37b durch die Pupille des Patientenauges auf die Retina fallen können. Außerdem müssen die Strahlengänge zu einem nennenswerten Teil durch den optisch aktiven Teil der implantierten Intraokularlinse 38 fallen, der typischerweise einen Durchmesser von 5-6 mm besitzt.

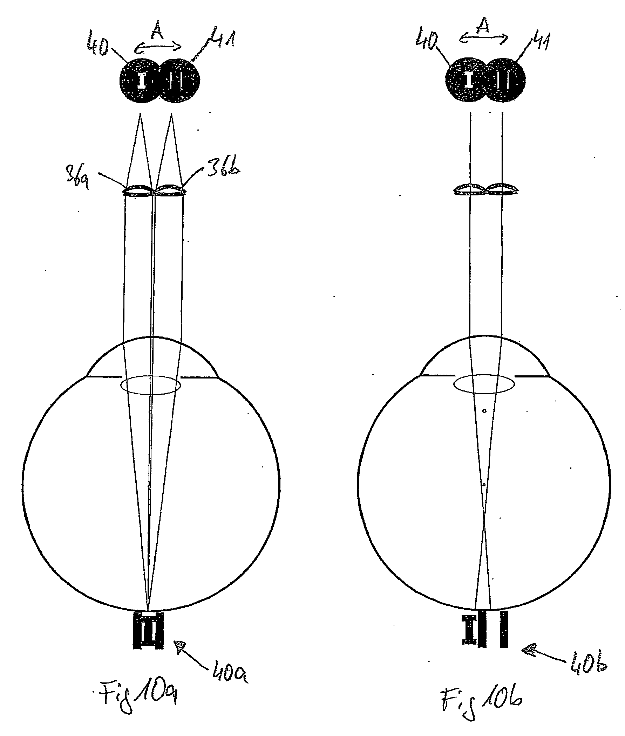

Die parallelen Strahlengänge 37a, 37b durchsetzen das Patientenauge und die Intraokularlinse 38 und fallen auf die Retina 19. Falls das Auge auf die Ferne korrigiert ist (Fig. 10a), treffen sich die Strahlengänge 37a, 37b wie im vorigen Beispiel auf der Retina und die beiden Mikrostrukturen 40, 41 werden zueinander zentriert 40a auf denselben Ort der Retina abgebildet. Ist das Patientenauge mit der implantierten Intraokularlinse 38 nicht auf unendlich eingestellt, liegt die Bildebene entweder vor oder hinter der Retina. Dies führt wie in der vorigen Ausführungsform dazu, dass die Bilder der Mikrostrukturen 40, 41 auf der Retina zueinander verschoben sind.

Selbstverständlich können auch mehr als zwei Mikrostrukturplatten und Objektive verwendet werden, die dann in analoger Weise eine Refraktionsmessung ermöglichen.

Das Modul ist mit einem Drehlager 43 unter der Frontlinse befestigt. Um die Refraktion in unterschiedlichen Meridianen des Auges zu prüfen, kann das optische Modul um die Achse 39 gedreht werden. Das Modul ist außerdem mit einem Drehgelenk 44 an dem Drehlager 43 befestigt und kann aus dem Strahlengang 8a, 8b, 9 des Operationsmikroskops herausgeschwenkt und in eine Parkposition gebracht werden.
Dies kann wieder manuell oder elektrisch geschehen.

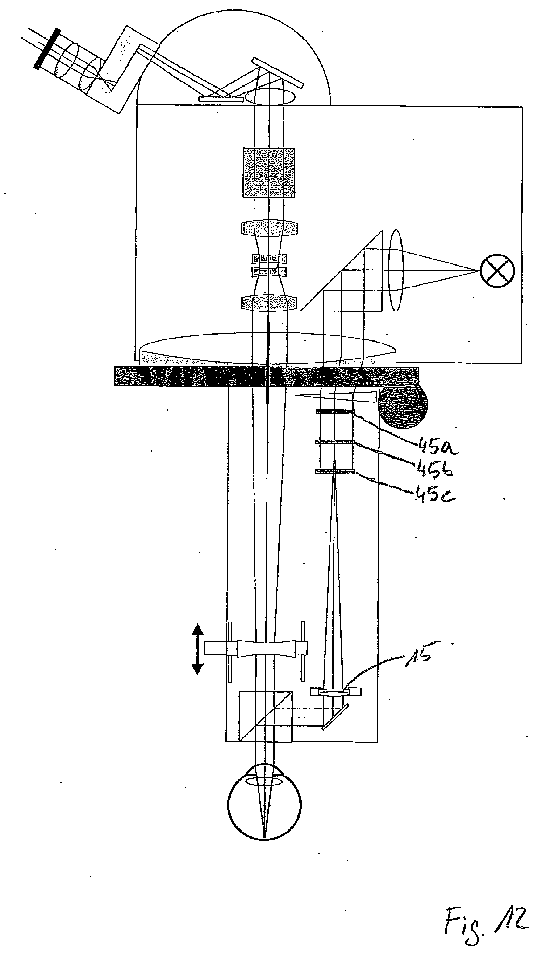

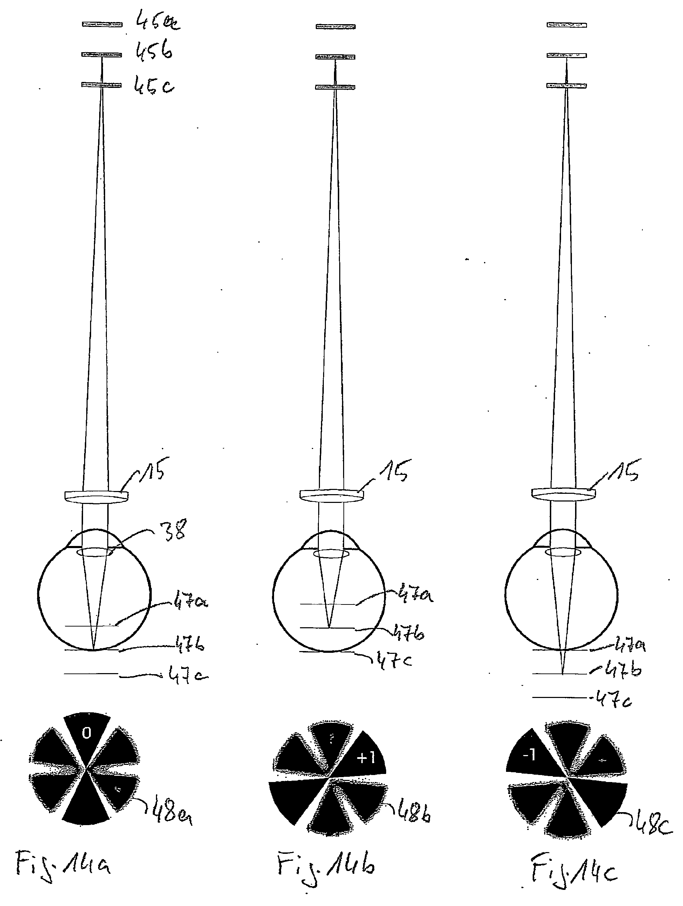

Die vierte Ausführungsform (Fig. 12) leitet sich aus der ersten ab. Jedoch verfügt das Modul 10 jetzt über ein ortsfestes Objektiv 15 und über mehr als eine, im konkreten Fall über drei ortsfeste Mikrostrukturplatten 45a, 45b, 45c, die in Abständen zueinander entlang der optischen Achse des Objektivs 15 angeordnet sind und deren Bildlagen im Patientenauge sich dadurch unterscheiden. Dabei ist beispielsweise die mittlere Platte 45b so positioniert, dass das Objektiv 15 die Mikrostruktur nach unendlich abbildet. Ein für die Ferne korrigiertes Patientenauge bildet die auf dieser Platte aufgebrachte Mikrostruktur mit der Bildlage 47b scharf auf die Retina ab Fig. 14a. Die Strukturen der davor (45c) und dahinter (45a) liegenden Mikrostrukturplatten werden daher unscharf abgebildet (48a), da sich ihre Bildlagen hinter der Retina (45c) oder vor der Retina (45a) befinden.

Ist der Patient nach Implantation der Intraokularlinse 38 kurzsichtig (Fig. 14b), d.h. die Brechkraft so stark, dass ein in der Ferne liegendes Objekt vor der Retina abgebildet wird, verschieben sich die Bildlagen 47a, 47b, 47c zum Objektiv 15 hin. Im konkreten Fall sei der Patient +1 dpt kurzsichtig. Durch entsprechende Wahl des Abstands der Mikrostrukturplatte 45c von der Strukturplatte 45b, z.B. durch Kalibrierung, befindet sich die Bildlage der Mikrostruktur 46c dann auf der Retina, die Mikrostruktur 46c wird also scharf auf die Retina abgebildet, während die Bilder der anderen Mikrostrukturen unscharf erscheinen 48b. Die Mikrostruktur 46c ist entsprechend der Kalibrierung beschriftet und die Beschriftung der jeweils am schärfsten abgebildeten Struktur in diesem Fall: "+1" zeigt dem Chirurgen den entsprechenden Refraktionsfehler an.

Ist der Patient hingegen nach Implantation der Intraokularlinse 38 weitsichtig (Fig. 14c), im konkreten Fall -ldpt, wird die Mikrostruktur 46a der Mikrostrukturplatte 45a scharf abgebildet, während die anderen Bilder unscharf sind 48c. Selbstverständlich sind andere Abstufungen als im konkreten Beispiel und die Verwendung von mehr Strichplatten möglich.

Die Mikrostrukturen 46a, 46b, 46c der Mikrostrukturplatten 45a, 45b, 45c sind so gewählt, dass sich ihre Bilder nicht überlagern. Dies ermöglicht ein komfortables Ablesen. Ihre fächerartige Anordnung dunkelt die Beleuchtung ab, um die Unschärfe der Kanten in dunklem Umfeld gut erkennen zu können.

## Patentansprüche

1. Operationsmikroskop für die Augenchirurgie mit einer Frontlinse (7), einem Vergrößerungssystem (4) und einem Einblick (1), die einen Beobachtungsstrahlengang (8a, 8b) definieren, der in der Augenlinsenebene (50) fokussierbar ist, wobei das Operationsmikroskop eine Abbildeanordnung (11, 15, 16, 17) und eine Brechungsanordnung (20) umfasst, die folgende Merkmale aufweisen:
a. für die Abbildeanordnung (11, 15, 16, 17) ist ein Aktivzustand und ein Passivzustand vorgesehen;
b. im Aktivzustand erzeugt die Abbildeanordnung (11, 15, 16, 17) eine Mehrzahl von Strukturabbildungen (12a, 12b, 40a, 40b), deren Strahlengänge (33a, 33b, 37a, 37b) den Beobachtungsstrahlengang (8a, 8b) überlagern und deren Strahlengänge (33a, 33b, 37a, 37b) parallel zueinander in der Augenlinsenebene (50) auftreffen;
c. im Passivzustand ist der Beobachtungsstrahlengang (8a, 8b) nicht durch die Abbildeanordnung (11, 15, 16, 17) beeinflusst;
d. für die Brechungsanordnung (20) ist ein Aktivzustand und ein Passivzustand vorgesehen;
e. im Aktivzustand wird der Beobachtungsstrahlengang (8a, 8b) durch die Brechungsanordnung (20) aufgeweitet;
f. im Passivzustand ist der Beobachtungsstrahlengang (8a, 8b) nicht durch die Brechungsanordnung (20) beeinflusst;

2. Operationsmikroskop nach Anspruch 1, **dadurch gekennzeichnet, dass** für die Abbildeanordnung (11, 15, 16, 17) ein Modus vorgesehen ist, in dem die Strukturabbildung (13a, 13b, 40a, 40b) in Form paralleler Strahlen auf die Augenlinsenebene (50) trifft.

3. Operationsmikroskop nach Anspruch 1 oder 2, **dadurch gekennzeichnet, dass** für die Brechungsanordnung (20) ein Modus vorgesehen ist, in dem ein auf die Augenlinsenebene (50) fokussierter Beobachtungsstrahlengang (8a, 8b) in einen parallelen Beobachtungsstrahlengang (8a, 8b) umgewandelt wird.

4. Operationsmikroskop nach einem der Ansprüche 1 bis 3, **dadurch gekennzeichnet, dass** die Abbildeanordnung (11, 15, 16, 17) und die Brechungsanordnung (20) in einem Zusatzmodul (10) des Operationsmikroskops zusammengefasst sind.

5. Operationsmikroskop nach Anspruch 4, **dadurch gekennzeichnet, dass** das Zusatzmodul (10) schwenkbar mit dem Operationsmikroskop verbunden ist.

6. Operationsmikroskop nach Anspruch 4 oder 5, **dadurch gekennzeichnet, dass** das Zusatzmodul (10) um die optische Achse der Frontlinse (7) drehbar ist.

7. Operationsmikroskop nach einem der Ansprüche 1 bis 6, **dadurch gekennzeichnet, dass** die Abbildeanordnung (11, 15, 16, 17) ein Objektiv (15) und ein Struktursubstrat (11) aufweist und dass im Aktivzustand das Objektiv (15) ein von dem Struktursubstrat (11) ausgehendes Strahlenbündel auf unendlich abbildet.

8. Operationsmikroskop nach einem der Ansprüche 1 bis 7, **dadurch gekennzeichnet, dass** das Operationsmikroskop eine Mikroskopbeleuchtung (6) umfasst und dass im Aktivzustand die Abbildeanordnung (11, 15, 16, 17) im Beleuchtungsstrahlengang der Mikroskopbeleuchtung (6) angeordnet ist.

9. Operationsmikroskop nach Anspruch 7 oder 8, **dadurch gekennzeichnet, dass** die Länge des Lichtwegs zwischen dem Struktursubstrat (11) und dem Objektiv (15) veränderbar ist.

10. Operationsmikroskop nach Anspruch 9, **dadurch gekennzeichnet, dass** eine Skala (27, 27a) vorgesehen ist, auf der die Längenveränderung des Lichtwegs ablesbar ist.

11. Operationsmikroskop nach einem der Ansprüche 7 bis 10, **dadurch gekennzeichnet, dass** die Abbildeanordnung (11, 15, 16, 17) eine Mehrzahl von Struktursubstraten (45a, 45b, 45c) umfasst, die in unterschiedlichen Abständen zum Objektiv (15) angeordnet sind.

12. Operationsmikroskop nach einem der Ansprüche 1 bis 11, **dadurch gekennzeichnet, dass** zur Unterscheidung der Strukturabbildungen (13a, 13b, 40a, 40b) ein Farbfilter (34a, 34b) vorgesehen ist.

13. Operationsmikroskop nach einem der Ansprüche 1 bis 12, **dadurch gekennzeichnet, dass** die Abbildeanordnung (11, 15, 16, 17) eine Blende (31) umfasst, mit der ein von dem Struktursubstrat (11) ausgehendes Strahlenbündel in eine Mehrzahl von Teilbündeln aufgespalten wird.

14. Operationsmikroskop nach einem der Ansprüche 1 bis 12, **dadurch gekennzeichnet, dass** die Abbildeanordnung (11, 15, 16, 17) ein erstes Objektiv (36a) und ein erstes Struktursubstrat (35a) zum Erzeugen einer ersten Strukturabbildung sowie ein zweites Objektiv (36b) und ein zweites Struktursubstrat (35b) zum Erzeugen einer zweiten Strukturabbildung umfasst.

## Claims

1. Operating microscope for eye surgery, comprising a front lens (7), a magnification system (4) and an observation end (1), which define an observation beam path (8a, 8b), which can be focussed in the eye lens plane (50), wherein the operating microscope includes a imaging arrangement (11, 15, 16, 17) and a refraction arrangement (20), which comprise the following features:
a. an active state and a passive state are provided for the imaging arrangement (11, 15, 16, 17);
b. in the active state, the imaging arrangement (11, 15, 16, 17) generates a plurality of structured images (12a, 12b, 40a, 40b), the beam paths (33a, 33b, 37a, 37b) of which are superimposed on the observation beam path (8a, 8b) and the beam paths (33a, 33b, 37a, 37b) of which impinge in parallel with one another in the eye lens plane (50);
c. in the passive state, the observation beam path (8a, 8b) is not affected by the imaging arrangement (11, 15, 16, 17);
d. an active state and a passive state are provided for the refraction arrangement (20);
e. in the active state, the observation beam path (8a, 8b) is widened by the refraction arrangement (20);
f. in the passive state, the observation beam path (8a, 8b) is not affected by the refraction arrangement (20).

2. Operating microscope according to claim 1, **characterised in that** a mode is provided for the imaging arrangement (11, 15, 16, 17) in which the structured image (13a, 13b, 40a, 40b) impinges on the eye lens plane (50) in the form of parallel beams.

3. Operating microscope according to either claim 1 or claim 2, **characterised in that** a mode is provided for the refraction arrangement (20) in which an observation beam path (8a, 8b) focussed on the eye lens plane (50) is converted into a parallel observation beam path (8a, 8b).

4. Operating microscope according to any of claims 1 to 3, **characterised in that** the imaging arrangement (11, 15, 16, 17) and the refraction arrangement (20) are grouped together in an additional module (10) of the operating microscope.

5. Operating microscope according to claim 4, **characterised in that** the additional module (10) is pivotally connected to the operating microscope.

6. Operating microscope according to either claim 4 or claim 5, **characterised in that** the additional module (10) can be rotated about the optical axis of the front lens (7).

7. Operating microscope according to any of claims 1 to 6, **characterised in that** the imaging arrangement (11, 15, 16, 17) comprises a lens (15) and a structural substrate (11) and **in that**, in the active state, the lens (15) images a beam bundle emanating from the structural substrate (11) to infinity.

8. Operating microscope according to any of claims 1 to 7, **characterised in that** the operating microscope comprises microscope illumination (6) and **in that**, in the active state, the imaging arrangement (11, 15, 16, 17) is arranged in the illumination beam path of the microscope illumination (6).

9. Operating microscope according to either claim 7 or claim 8, **characterised in that** the length of the light path between the structural substrate (11) and the lens (15) can be changed.

10. Operating microscope according to claim 9, **characterised in that** a scale (27, 27a) is provided on which the change in length of the light path can be read.

11. Operating microscope according to any of claims 7 to 10, **characterised in that** the imaging arrangement (11, 15, 16, 17) comprises a plurality of structural substrates (45a, 45b, 45c) which are arranged at different spacings from the lens (15).

12. Operating microscope according to any of claims 1 to 11, **characterised in that** a colour filter (34a, 34b) is provided to differentiate between the structured images (13a, 13b, 40a,40b).

13. Operating microscope according to any of claims 1 to 12, **characterised in that** the imaging arrangement (11, 15, 16, 17) comprises a shutter (31) by means of which one of the beam bundles emanating from the structural substrate (11) is split into a plurality of partial bundles.

14. Operating microscope according to any of claims 1 to 12, **characterised in that** the imaging arrangement (11, 15, 16, 17) comprises a first lens (36a) and a first structural substrate (35a) for producing a first structured image and a second lens (36b) and a second structural substrate (35b) for producing a second structured image.

## Revendications

1. Microscope d'opération pour la chirurgie oculaire avec une lentille frontale (7), un système d'agrandissement (4) et un aperçu (1), qui définissent un trajet de faisceaux d'observation (8a, 8b), qui peut être focalisé dans le plan du cristallin (50), le microscope d'opération comprenant un dispositif de représentation (11, 15, 16, 17) et un dispositif de réfraction (20), qui présentent les caractéristiques suivantes :
a) pour le dispositif de représentation (11, 15, 16, 17), un état actif et un état passif sont prévus ;
b) dans l'état actif, le dispositif de représentation (11, 15, 16, 17) produit une pluralité de représentations de structures (12a, 12b, 40a, 40b), dont les trajets de faisceaux (33a, 33b, 37a, 37b) se superposent avec le trajet de faisceaux d'observation (8a, 8b) et dont les trajets de faisceaux (33a, 33b, 37a, 37b) arrivent parallèles entre eux dans le plan du cristallin (50) ;
c) dans l'état passif, le trajet de faisceaux d'observation (8a, 8b) n'est pas influencé par le dispositif de représentation (11, 15, 16, 17) ;
d) pour le dispositif de réfraction (20) est état actif et un état passif sont prévus ;
e) dans l'état actif, le trajet de faisceaux d'observation (8a, 8b) est élargi par le dispositif de réfraction (20) ;
f) dans l'état passif, le trajet de faisceaux d'observation (8a, 8b) n'est pas influencé par le dispositif de réfraction (20).

2. Microscope d'opération selon la revendication 1, **caractérisé en ce que**, pour le dispositif de représentation (11, 15, 16, 17), un mode est prévu, dans lequel la représentation de la structure (13a, 13b, 40a, 40b) arrive sur le plan du cristallin (50) sous la forme de faisceaux parallèles.

3. Microscope d'opération selon la revendication 1 ou 2, **caractérisé en ce que**, pour le dispositif de réfraction (20), un mode est prévu, dans lequel un trajet de faisceaux d'observation (8a, 8b), focalisé sur le plan du cristallin (50), est transformé en un trajet de faisceaux d'observation (8a, 8b) parallèle.

4. Microscope d'opération selon l'une des revendications 1 à 3, **caractérisé en ce que** le dispositif de représentation (11, 15, 16, 17) et le dispositif de réfraction (20) sont regroupés dans un module supplémentaire (10) du microscope d'opération.

5. Microscope d'opération selon la revendication 4, **caractérisé en ce que** le module supplémentaire (10) est relié de manière pivotante avec le microscope d'opération.

6. Microscope d'opération selon la revendication 4 ou 5, **caractérisé en ce que** le module supplémentaire (10) est rotatif autour de l'axe optique de la lentille frontale (7).

7. Microscope d'opération selon l'une des revendications 1 à 6, **caractérisé en ce que** le dispositif de représentation (11, 15, 16, 17) comprend un objectif (15) et un substrat structuré (11) et **en ce que**, dans l'état actif, l'objectif (15) représente un faisceau sortant du substrat structuré (11) vers l'infini.

8. Microscope d'opération selon l'une des revendications 1 à 7, **caractérisé en ce que** le microscope d'opération comprend un éclairage de microscope (6) et **en ce que**, dans l'état actif, le dispositif de représentation (11, 15, 16, 17) est disposé dans le trajet de faisceau d'éclairage de l'éclairage du microscope (6).

9. Microscope d'opération selon la revendication 7 ou 8, **caractérisé en ce que** la longueur du trajet lumineux entre le substrat structuré (11) et l'objectif (15) peut être modifiée.

10. Microscope d'opération selon la revendication 9, **caractérisé en ce qu'**une échelle (27, 27a) est prévue, sur laquelle la modification de longueur du trajet lumineux peut être lue.

11. Microscope d'opération selon l'une des revendications 7 à 10, **caractérisé en ce que** le dispositif de représentation (11, 15, 16, 17) comprend une pluralité de substrats structurés (45a, 45b, 45c) qui sont disposés à des distances différentes de l'objectif (15).

12. Microscope d'opération selon l'une des revendications 1 à 11, **caractérisé en ce que**, pour la différentiation des représentations de structures (13a, 13b, 40a, 40b), un filtre de couleur (34a, 34b) est prévu.

13. Microscope d'opération selon l'une des revendications 1 à 12, **caractérisé en ce que** le dispositif de représentation (11, 15, 16, 17) comprend un diaphragme (31) avec lequel un faisceau sortant du substrat structuré (11) est divisé en une pluralité de faisceaux partiels.

14. Microscope d'opération selon l'une des revendications 1 à 12, **caractérisé en ce que** le dispositif de représentation (11, 15, 16, 17) comprend un premier objectif (36a) et un premier substrat structuré (35a) pour la production d'une première représentation de structure ainsi qu'un deuxième objectif (36b) et un deuxième substrat structuré (35b) pour la production d'une deuxième représentation de structure.
